# EUROPEAN PATENT APPLICATION

(11) **EP 4 821 443 A1**
(43) Date of publication of application: **14.10.2026**
(21) Application number: 26170500.8
(22) Date of filing: 02.04.2026
(51) Int. Cl.: A61B 7/00, A61B 7/02, A61B 7/04

(54) **CHESTPIECE, METHOD AND AUSCULTATION SYSTEM**

(30) Priority: 08.04.2025 FI 20253125
(71) Applicant: Lapsi Health Holding B.V., 1098 XH Amsterdam (NL)
(72) Inventor: Bringas Dimitriades, Jhonatan, 1098 XH Amsterdam (NL); van Stijn, Diana, 1098 XH Amsterdam (NL)
(74) Representative: Primrose Oy

(57) **Abstract**

The invention relates to a chestpiece, a method an auscultation system. The chestpiece (10) comprises a first sound capture module (15, 20, 30) in connection with the body (12), the first sound capture module (15, 20, 30) comprising a contact surface (14, 15) for contact with a patient body, and being configured to capture internal body sounds in contact with the patient body via the contact surface (14 15) and to convert the internal body sounds into an internal body sound signal, and a second sound capture module (32) in connection with the body (12), the second sound capture module (32) being configured to capture ambient sounds from ambient space of the chestpiece (10) and to convert the ambient sounds into an ambient sound signal. The second sound capture module (32) comprises a microphone array comprising three of more ambient microphones (37).

## Description

### FIELD OF THE INVENTION

The present invention relates to a chestpiece and more particularly to a chestpiece according to preamble of claim 1. The present invention further relates to a method for sound analysis and more particularly to a method according to preamble of claim 5. The present invention also relates to an auscultation system and more particularly to an auscultation system according to preamble of claim 11.

### BACKGROUND OF THE INVENTION

In the prior art auscultation is carried out by doctors using stethoscopes.

The conventional auscultation provides momentary information of the health condition of internal organs of the patient. However, long-term health information cannot be obtained. Further, the conventional auscultation is restricted to information which is obtained based on internal sounds of the patient during the auscultation.

### BRIEF DESCRIPTION OF THE INVENTION

An object of the present invention is to provide a chestpiece, a method and an auscultation system so as to overcome or at least alleviate the prior art disadvantages.

The objects of the invention are achieved by a chestpiece which is characterized by what is stated in the independent claim 1. The objects of the invention are further achieved by a method which is characterized by what is stated in the independent claim 5. The objects of the invention are also achieved by a n auscultation system which is characterized by what is stated in the independent claim 11.

The preferred embodiments of the invention are disclosed in the dependent claims.

The invention is based on the idea of providing a chestpiece for auscultation and sound analysis. The chestpiece comprises a body, a first sound capture module in connection with the body, the first sound capture module comprising a contact surface for contact with a patient body, and being configured to capture internal body sounds in contact with the patient body via the contact surface and to convert the internal body sounds into an internal body sound signal, a second sound capture module in connection with the body, the second sound capture module being configured to capture ambient sounds from ambient space of the chestpiece and to convert the ambient sounds into an ambient sound signal, and a processing unit for operating the chestpiece. According to the invention the second sound capture module comprises three of more ambient microphones arranged spaced apart from each other and configured to capture ambient sounds from ambient space of the chestpiece and to convert the ambient sounds into the ambient sound signals.

In some embodiments, the second sound capture module comprises three or more separate ambient microphones arranged spaced apart from each other and configured to capture ambient sounds from ambient space of the chestpiece and to convert the ambient sounds into the ambient sound signals.

The three or more separate ambient microphones enable efficient capture of ambient sounds.

In some other embodiments, the second sound capture module comprises six or more separate ambient microphones arranged spaced apart from each other and configured to capture ambient sounds from ambient space of the chestpiece and to convert the ambient sounds into the ambient sound signals.

The six or more separate ambient microphones enable efficient and detailed capture of ambient sounds from different directions.

In some embodiments, the second sound capture module comprises a microphone array comprising three of more ambient microphones arranged spaced apart from each other and configured to capture ambient sounds from ambient space of the chestpiece and to convert the ambient sounds into the ambient sound signals.

The microphone array is efficient in capturing human originating sounds from ambient of the chestpiece and enables extracting audio features from the ambient sound signals generated by the ambient microphones of the microphone array.

In the context of this application the auscultation sound signal comprises the output signal of the first sound capture module or the first microphone thereof or auscultation signal data derived from the auscultation sound signal.

In the context of this application the ambient sound signal comprises the output signal of the second sound capture module or the ambient microphones thereof or ambient signal data derived from the ambient sound signal.

In some embodiments, the microphone array comprises six or more ambient microphones arranged spaced apart from each other and configured to capture ambient sounds from ambient space of the chestpiece and to convert the ambient sounds into the ambient sound signals.

Six or more ambient microphones enables enhanced sound capture from ambient of the chestpiece and from all directions of the chestpiece.

The ambient microphones, separate microphones or the microphones of the microphone array, may be micro-electromechanical microphones (MEMs microphones), electret condenser microphones, or the like microphone.

The three or more ambient microphones comprise omnidirectional microphones or directional microphones. Alternatively, the three or more ambient microphones comprise both omnidirectional and directional microphones.

Accordingly, ambient sounds may be captured efficiently from different directions.

The directional microphones are directed to different directions relative to each other for capturing ambient sounds from different directions.

In some embodiments, the body comprises ambient sound openings open to the ambient space of the chestpiece, and the ambient are arranged in connection with the ambient sound openings, respectively.

The ambient sound openings enhance capturing high-frequency sounds and human originating sounds with the ambient microphones.

In some other embodiments, the body comprises ambient acoustic chambers inside the body, the ambient acoustic chambers comprise an ambient sound opening open to the ambient space of the chestpiece, and the ambient microphones are arranged in connection with the ambient acoustic chambers, respectively.

The ambient acoustic chamber provides amplification of the ambient sounds captured by the ambient microphones.

In some embodiments, the ambient sound openings are provided with an acoustic filter element arranged to cover the ambient sound opening.

The acoustic filter element is arranged to reduce environmental noise, wind interference, and the risk of contamination.

The acoustic filter element is a mechanical filter element provided to the acoustic opening or in connection of the acoustic opening.

In some embodiments, the ambient microphones, and/or the ambient sound openings, and/or the ambient acoustic chambers are arranged in connection with a top surface of the body opposite to the contact surface, and the sound openings are provided to the top surface.

The top surface enables efficient ambient sound capturing from different directions when the chestpiece is placed on the support surface such that the contact surface is towards the support surface.

In some embodiments, the processing unit is configured to selectively operate the chestpiece in an auscultation mode and in an ambient mode, and that
in the auscultation mode:
   the first sound capture module is configured to capture internal body sounds of the patient body in contact with the patient body via the contact surface and to convert the internal body sounds into an internal body sound signal; and
in the ambient mode:
   the second sound capture module is configured to capture ambient sounds from the ambient space of the chestpiece and to convert the ambient sounds into the ambient sound signals.

In some embodiments, in the auscultation mode the second sound capture module is configured to capture ambient sounds from the ambient space of the chestpiece and to convert the ambient sounds into the ambient sound signals and ambient sound signals are utilized for noise cancellation during the auscultation.

In some embodiments, in the ambient mode operation of the first sound capture module is disabled such that first sound capture module is disabled to capture sounds.

The above features concerning the auscultation mode and the ambient mode may also be provided in same embodiment.

In some embodiments, the microphone array comprises a beamforming module configured combine the ambient sound signals of the three of more ambient microphones into a single ambient sound signal outputted from the microphone array.

The beamforming enhances directional focus on the sound, enables reducing background noise and enhance voice clarity.

The present invention is further based on an idea of providing a method for auscultation and sound analysis. The method utilizing a chestpiece for auscultation, the chestpiece comprising a body, a first sound capture module in connection with the body, the first sound capture module comprising a contact surface for contact with a patient body, and being configured to capture internal body sounds in contact with the patient body via the contact surface and to convert the internal body sounds into an internal body sound signal, and a second sound capture module in connection with the body, the second sound capture module being configured to capture ambient sounds from ambient space of the chestpiece and to convert the ambient sounds into an ambient sound signal.

The method is executed using hardware and software resources of computing device, the method causing the computing device to perform operations comprising:
- obtaining the ambient sound signal from the second sound capture module,
- carrying out audio feature extraction to the ambient sound signal to obtain extracted audio features from the ambient sound signal,
- generating audio feature data of the ambient sound signal based on the extracted audio features, and
- obtaining a sound characteristic output by processing the audio feature data of the ambient sound signal.

The method enables utilizing ambient sounds captured by the second sound capture module for analysing health of a patient by processing the captured ambient sounds.

In some embodiments, carrying out audio feature extraction to the ambient sound signal(s) to obtain extracted audio features from the ambient sound signal(s) comprises one or more of the following:
- time-domain feature extraction configured to extract intensity features of the ambient sound signal over time,
- frequency-domain feature extraction configured to extracting frequency features of the ambient sound signal,
- cepstral feature extraction configured to extract vocal track shape features of the ambient sound signal,
- voice quality feature extraction configured to extract consistency or smoothness or consistency and smoothness features of the ambient sound signal,
- temporal feature extraction configured to extract pattern features of the ambient sound signal, and
- prosodic feature extraction configured to extract expressiveness features of the ambient sound signal.

The different extracted sound features may be utilized for analysing different health related issues by processing the captured ambient sounds.

In some embodiments, the method comprises
- determining human originating sound components in the ambient sound signal,
- carrying out the audio feature extraction to the detected human originating sound components to obtain extracted audio features from the detected human originating sound components,
- generating the audio feature data of the detected human originating sound components based on the extracted audio features, and
- obtaining the sound characteristic output by processing the audio feature data of the detected human originating sound component.

Determining or identifying the human originating sound components or segments in the ambient sound signal enables processing only the determined human originating sound components.

In some embodiments, determining human originating sound components in the ambient sound signal comprises one or more of the following:
- identifying speech sound components in the ambient sound signal, the speed sound components representing sound components originating from human speech, or
- identifying cough sound components in the ambient sound signal, the cough sound components representing sound components originating from human cough, or
- identifying external body sound components in the ambient sound signal, the external body sound components representing sound components originating from human body; or
- identifying breathing sound components in the ambient sound signal, the breathing sound components representing sound components originating from human breathing.

Identifying speech, cough, breathing and other external body sound components enables isolating them from each other and processing separately with at least partly different feature extraction algorithms.

In some embodiments, obtaining the sound characteristic output comprises obtaining speech analysis output by processing the audio feature data generated by carrying out one or more of the following: prosodic feature extraction, voice quality feature extraction, a temporal feature extraction for health monitoring of a patient.

In some other embodiments, obtaining the sound characteristic output comprises obtaining speech analysis output by processing the audio feature data generated by carrying out prosodic feature extraction and voice quality feature extraction for emotional state monitoring of a patient.

In some further embodiments, obtaining the sound characteristic output comprises obtaining speech analysis output by processing the audio feature data generated by carrying out temporal feature extraction for neurological health monitoring of a patient.

In some embodiments, the method comprises carrying out speech recognition to the ambient sound signal to obtain speech data based on the ambient sound signal, carrying out speech-to-text processing to generate text data based on the speech data, and obtaining a text output by processing the text data.

Accordingly, the chestpiece and ambient microphones thereof are utilized for speech recognition and speech-to-voice processing. This enables generating text data and further text output based on the captured ambient sounds.

Therefore, the auscultation system may be further utilized for generating doctor-patient consultation summaries and transcriptions. The text data or output text may be further utilized as an input to a text processing algorithm, such as machine learning algorithm, neural network, large language model or the like.

In some embodiments, the method comprises carrying out speech recognition to the ambient sound signal to obtain speech data based on the ambient sound signal, generating voice control data based on the speech data, the voice control data comprising identified control commands for operating hardware or software resources, and obtaining a control command output by processing the voice data.

This enables operating the chestpiece, the auscultation system, or external devices connected to the chestpiece or the auscultation system by utilizing voice command.

In some embodiments, the method is configured to generate chestpiece control data as the voice control data based on speech data and obtain chestpiece control command output. The chestpiece control command output being configured to generate auscultation mode selection data and ambient mode selection mode data, respectively, based on the speech data to select auscultation mode or ambient mode of the chestpiece.

In some embodiments, obtaining the sound characteristic output comprises obtaining cough analysis output by processing the audio feature data generated by carrying out one or more of the following: time-domain feature extraction, frequency-domain feature extraction and cepstral feature extraction for cough monitoring of a patient.

In some other embodiments, obtaining the sound characteristic output comprises obtaining cough analysis output by processing the audio feature data generated by carrying out frequency-domain feature extraction and cepstral feature extraction for cough classification of patient.

In some further embodiments, obtaining the sound characteristic output comprises obtaining cough analysis output by processing the audio feature data generated by carrying out time-domain feature extraction for cough characteristics monitoring.

In some embodiments, obtaining the sound characteristic output comprises obtaining breathing analysis output by processing the audio feature data generated by carrying out one or more of the following: time-domain feature extraction, frequency-domain feature extraction, voice quality feature extraction and temporal feature extraction for breathing monitoring of a patient.

In some other embodiments, obtaining the sound characteristic output comprises obtaining breathing analysis output by processing the audio feature data generated by carrying out frequency-domain feature extraction and temporal feature extraction for cough classification of patient; or

In some further embodiments, obtaining the sound characteristic output comprises obtaining breathing analysis output by processing the audio feature data generated by carrying out time-domain feature extraction for breathing characteristics monitoring.

The method comprises obtaining a breathing analysis output based on processing the audio feature data. The breathing analysis output may be further utilized for analysing sleep or sleep tracking a person or patient via sound analysis. Accordingly, the method enables sleep analysis and sleep tracking by capturing the ambient sound signal with the second sound capture module of the stethoscope.

The method is preferably carried out by utilizing the chestpiece as disclosed above.

The method is also preferably carried out by utilizing an auscultation system as disclosed below.

The present invention is further based on an idea of providing an auscultation system for auscultation and sound analysis. The auscultation system comprises a chestpiece for auscultation and an ambient sound processing module. The chestpiece comprises a body, a first sound capture module in connection with the body, the first sound capture module comprising a contact surface for contact with a patient body, and being configured to capture internal body sounds in contact with the patient body via the contact surface and to convert the internal body sounds into an internal body sound signal, a second sound capture module in connection with the body, the second sound capture module being configured to capture ambient sounds from ambient space of the chestpiece and to convert the ambient sounds into an ambient sound signal, and a processing unit for operating the chestpiece.

According to the invention the second sound capture module comprises a microphone array comprising three of more ambient microphones arranged spaced apart from each other and configured to capture ambient sounds from ambient space of the chestpiece and to convert the ambient sounds into the ambient sound signals.

Further, according to the invention the ambient sound processing module configured to perform processing of the ambient sound signals, the processing comprising:
obtaining the ambient sound signal from the second sound capture module,
carrying out audio feature extraction to the ambient sound signal to obtain extracted audio features from the ambient sound signal,
generating audio feature data of the ambient sound signal based on the extracted audio features, and
obtaining a sound characteristic output by processing the audio feature data of the ambient sound signal.

The auscultation system further comprises an output unit configured to provide the sound characteristic output as an ambient sound analysis output.

In some embodiments, the processing unit is configured to selectively operate the chestpiece in an auscultation mode and in an ambient mode. In the auscultation mode the first sound capture module is configured to capture internal body sounds of the patient body in contact with the patient body via the contact surface and to convert the internal body sounds into an internal body sound signal, and in the ambient mode the second sound capture module is configured to capture ambient sounds from the ambient space of the chestpiece and to convert the ambient sounds into the ambient sound signal, and the ambient sound processing module configured to perform the processing of the ambient sound signals.

In some embodiments, the chestpiece comprises a movement sensor operatively connected to the processing unit, and the processing unit is configured to initiate the ambient mode as response to detecting immobility of the chestpiece, or initiate the ambient mode as response to detecting immobility of the chestpiece a pre-determined immobility time period.

In some other embodiments, the chestpiece comprises a movement sensor operatively connected to the processing unit, and the processing unit is configured to terminate the ambient mode as response to detecting movement of the chestpiece.

In some further embodiments, the chestpiece comprises a movement sensor operatively connected to the processing unit, and the processing unit is configured to initiate the ambient mode as response to detecting immobility of the chestpiece, or initiate the ambient mode as response to detecting immobility of the chestpiece a pre-determined immobility time period and to terminate the ambient mode as response to detecting movement of the chestpiece.

In some embodiments, the ambient sound processing module is provided to the chestpiece, and operatively connected to the processing unit or provided in connection with processing unit.

In some other embodiments, the ambient sound processing module is provided to an external computing device configured to receive the ambient sound signal from the chestpiece.

In some further embodiments, the ambient sound processing module is provided as a distributed module configured to distribute the processing of the ambient sound signal between the chestpiece and one or more external computing devices.

In some embodiments, obtaining the sound characteristic output by the processing module comprises generating a visual or audio output data, the sound characteristic output comprising the visual or audio output data.

In some embodiments of the auscultation system, the chestpiece is a chestpiece as disclosed above.

In some embodiments, the auscultation system is configured to carry out the method as disclosed above.

The present invention is further based on an idea of providing a computer program product comprising computer instructions in at least one memory which, when executed by at least one processor, cause a computing apparatus to perform the method as disclosed above, or the method of the ambient sound processing module.

The present invention enables utilizing the chestpiece of the electronic stethoscope system of the auscultation system for passive health diagnosis of a patent based external body sounds, such as speech, coughs or other external sounds, captured by the chestpiece of the stethoscope. This enables personalized long-term health motoring in a non-invasive manner.

### BRIEF DESCRIPTION OF THE DRAWINGS

The invention is described in detail by means of specific embodiments with reference to the enclosed drawings, in which
Figures 1 and 2 show schematically outer views of the chestpiece of an electronic stethoscope.
Figure 3 and 4 show schematically sound capture modules of a chestpiece;
Figure 5 shows schematically a cross-sectional view of a chestpiece;
Figure 6 shows schematically a processing unit of a chestpiece;
Figures 7 and 8 show schematically auscultation mode and ambient mode of a chestpiece;
Figure 9 shows schematically an auscultation system in auscultation mode;
Figure 10 shows schematically an auscultation system in ambient mode; and
Figure 11 shows schematically an ambient sound processing module.

### DETAILED DESCRIPTION OF THE INVENTION

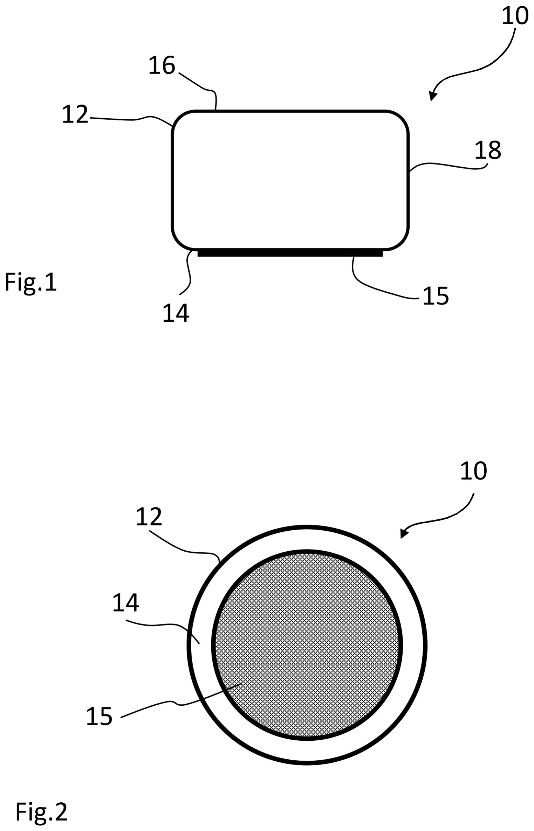

Figure 1 shows schematically a side view of one embodiment of a chestpiece 10 for an electronic stethoscope or an auscultation system. The chestpiece 10 is configured to provide auscultated sounds of a patient body, for example heart, lungs, bowel, arteries and veins.

The chestpiece 10 comprises a body 12. The body comprises an outer top surface 16, an outer front surface 14 and outer side surface(s) 18 extending between the outer top surface 16 and the outer front surface 14. The outer surfaces define a chestpiece inner space 11 inside the chestpiece 10. The outer front surface 14 is configured to be placed against a patient body or skin during health examination. The outer front surface 14 is configured as a contact surface for contact with the patient body.

The outer front surface 14 is provided with a diaphragm 15 forming at least part of the outer front surface 14 of the body 12.

Figure 2 shows schematically an end view of the chestpiece 10 in a direction towards the outer front surface 14. The diaphragm 15, or membrane, is provided to the outer front surface 14.

The chestpiece 10 has circular cylinder shape, as shown in figures 1 and 2.

It should be noted, that the chestpiece 10 may also have a different three-dimensional shape, such as oval cylindrical shape, or rectangular or polygonal cylindrical shape.

Further it should be noted that the diaphragm 15 may be omitted in some embodiments.

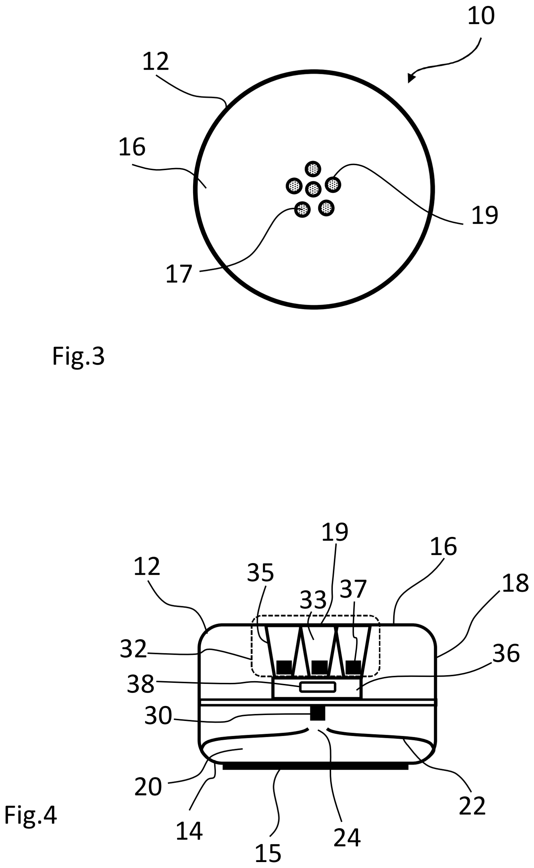

Figure 3 shows schematically a cross another end view of the chestpiece 10 in a direction towards the top surface 16. The top surface is provided ambient sound openings 19. The top surface 16 may be provided with one or more ambient sound openings 19.

It should be noted that the ambient sound openings 19 are preferably provided to the top surface 16 for efficiently capturing ambient sounds when the chestpiece 10 is placed on a support surface such that the outer front surface is towards the support surface.

However, it should be noted that the ambient sound opening(s) 19 may be provided to the side surface(s) 18 of the chestpiece 10. Further alternatively, the ambient sound openings 19 may be provided to the top surface 16 and the side surface(s) 18 of the chestpiece 10.

Figure 4 shows schematically a cross-sectional view of the chestpiece 10.

The chestpiece 10 comprises a first sound capture module in connection with the body 12. The first sound capture module comprises the contact surface 14, 15 for contact with the patient body. The first sound capture module further comprises a first sound capture device 30 configured to capture internal body sounds in contact with the patient body via the contact surface 14, 15 and to convert the internal body sounds into an internal body sound signal.

The first sound capture device 30 comprises a microphone, such as a MEMS (microphone micro-electromechanical) microphone.

As shown in figure 4, the first sound capture module of the chestpiece 10 comprises an audio chamber 20 inside the body 12. The audio chamber 20 is defined by audio chamber walls 22 and the diaphragm 15. The diaphragm 15 is arranged to separate the audio chamber 20 form the outside of the chestpiece 10 at the outer front surface 14. Thus, the audio chamber 20 is provided in connection with the outer front surface 14.

The diaphragm 15 has a planar shape.

The diaphragm 15 extends parallel to the outer front surface 14.

When the outer front surface 14 is placed against patient body, the diaphragm 15 is also placed against the patient body. The diaphragm 15 is provided as a flexible diaphragm. Flexibility of the diaphragm 15 enables the diaphragm 15 to move or vibrate as a response to body sounds of the patient when the outer front surface 14 and the diaphragm 15 is placed against the patient body. The movement or vibration of the diaphragm 15 generates acoustic pressure waves into the audio chamber 20.

The audio chamber 20 has bell-shaped form, as shown schematically in figure 4. The diaphragm 15 or the outer front surface 14 is arranged to provide a bottom of the bell-shaped form. The end 24 of the bell-shaped audio chamber 20 is provided opposite the diaphragm 15. The bell-shaped form of the audio chamber 20 provides mechanical amplification of sound, sound waves or pressure waves generated with the diaphragm 15.

It should be noted that that the audio chamber 20 may also have a different shape. The audio chamber 20 may for example have a tapering or converging shape towards the top end 24 of the audio chamber 20. The top end 24 is arranged opposite the diaphragm 15.

The shape of the audio chamber 20 is arranged to provide amplification of the auscultated sounds towards the top end 24 of the audio chamber 20.

Further, it should be noted that in some embodiments the diaphragm 15 may be omitted. Thus, the audio chamber 20 is open to the outer front wall 14 of the chestpiece 10.

Further, it should be noted that that the audio chamber 20 may also omitted in some embodiments.

The chestpiece 10 comprises the first microphone 30 arranged in connection with the audio chamber 20. The first microphone is configured to capture auscultated sounds or the pressure waves provided by the diaphragm 15 and the audio chamber 20, and further convert the auscultated sounds into an auscultated sound signal.

The auscultated sound signal generated by the first microphone 30 is an electronic auscultated sound signal.

The first microphone 30 is arranged to in connection with the audio chamber 20 such that the first microphone 30 is arranged to capture the auscultated sounds from the audio chamber 20.

The first microphone 30 is arranged inside the audio chamber 20. Alternatively, the first microphone 30 is arranged to the audio chamber walls 22.

In the embodiment of figure 4, the first microphone 30 is arranged in connection with the top end 24 of the audio chamber 20.

The first microphone 30 is arranged to the top end 24 of the audio chamber 20.

In the embodiment of figure 4, the top end 24 of the audio chamber 20 comprises sound opening and the first microphone 30 is arranged to or in connection with the sound opening of the top end 24.

In some other embodiments the first microphone 30 is arranged to form the top end 24 of the audio chamber 20.

The first microphone 30 may be any kind of microphone device, such as the MEMS microphone.

Accordingly, the first sound capture module comprises the first microphone 30, the diaphragm 15 and the audio chamber 20.

The first sound capture module is configured to capture internal body sounds in contact with the patient body via the contact surface 14 and to convert the internal body sounds into an internal body sound signal.

As shown in figure 4, the chestpiece 10 comprises a second sound capture module 32 in connection with the body 12. The second sound capture module 32 is configured to capture ambient sounds from ambient space of the chestpiece 10 and to convert the ambient sounds into an ambient sound signal.

The second sound capture module 32 comprises a microphone array comprising three of more ambient microphones 37 arranged spaced apart from each other and configured to capture ambient sounds from ambient space of the chestpiece 10 and to convert the ambient sounds into the ambient sound signals.

In preferred embodiments, the microphone array comprises six or more ambient microphones 37 arranged spaced apart from each other and configured to capture ambient sounds from ambient space of the chestpiece 10 and to convert the ambient sounds into the ambient sound signals.

The microphone array comprises omnidirectional microphones 37 or directional microphones 37. Alternatively, the microphone array comprises both omnidirectional and directional microphones 37.

The second sound capture module 32 further comprises the ambient sound openings 19 open to the ambient space of the chestpiece 10. The ambient microphones 37 of the microphone array are arranged in connection with the ambient sound openings 19.

As shown in figure 4 there is one microphone 37 arranged in connection with each of the ambient sound openings 19.

The microphone 37 are directly aligned with the ambient sound openings 19 and towards the sound openings 19.

The ambient sound openings have a diameter between 3-5 mm. The diameter of 3 to 5 mm is arranged to focus on human vocal frequencies (300 Hz to 4 kHz) while balancing adequate sound capture with noise reduction.

The second sound capture module 32 further comprises ambient acoustic chambers 33 inside the body 12. The ambient acoustic chamber 33 comprise the ambient sound opening 19 open to the ambient space of the chestpiece 10. The ambient acoustic chamber 33 is arranged extend from the ambient sound opening 19 into body 12 of the chestpiece 10.

As shown in figure 4 there is one microphone 37 arranged in connection with each of the ambient acoustic chambers 33.

The ambient microphone 37 is arranged in connection with the ambient acoustic chambers 33.

As shown in figure4, the ambient microphone 37 is arranged inside the ambient acoustic chamber 33 directly aligned and opposite the ambient sound opening 19.

The ambient acoustic chamber 33 is formed by ambient acoustic chamber walls 35. The ambient acoustic chamber walls 35 are arranged to define ambient acoustic chamber 33 having a tapering or converging shape from the ambient sound opening towards ambient microphone 37. This provides amplification of the ambient sounds.

The ambient sound openings 19 are provided with an acoustic filter element 17 arranged to cover the ambient sound opening 19.

In some embodiments, the acoustic filter element 19 is provided as an acoustic mesh filter or an acoustic foam filter.

The acoustic filter element 17 is arranged to reduce environmental noise, wind interference, and the risk of contamination.

As shown in figure 4, the second sound capture module, the ambient microphones 37 of the microphone array, the ambient sound openings 19 and the ambient acoustic chambers 33 are arranged in connection with a top surface 16 of the body 12 opposite to the contact surface 14.

The microphone array of the second sound capture module 32 comprises a beamforming module 38 configured combine the ambient sound signals of the ambient microphones 37 of the microphone array into a single ambient sound signal outputted from the microphone array of the second sound capture module 32.

The beamforming module 38 may comprise spatial filter in into which the multiple ambient sound signals from the ambient microphones 37 of the microphone array are inputted, and the spatial filter is configured to combine the multiple ambient sound signals into a single ambient sound signal. In some embodiments, the single ambient sound signal is calculated by filtering each ambient sound signal of the ambient microphones through a digital FIR filter (finite impulse response filter) and summing the output of all filters.

It should be noted that beamforming module 38 may be provided as any kind beamforming module configured to combine the ambient sound signals of the ambient microphones 37 of the microphone array into a single ambient sound signal.

The beamforming module 38 may also be omitted.

The chestpiece 10 is further provided with a damping element 36. The second sound capture module 32 and the microphone array thereof is supported to the damping element 36 in the chestpiece 10. The damping element may be kind of damping element arranged to prevent or at least decrease vibrations of the second sound capture module 32 or the microphone array.

In some embodiment, the damping element 36 is soft or resilient element. The damping element 36 may be made of a rubber or foam material.

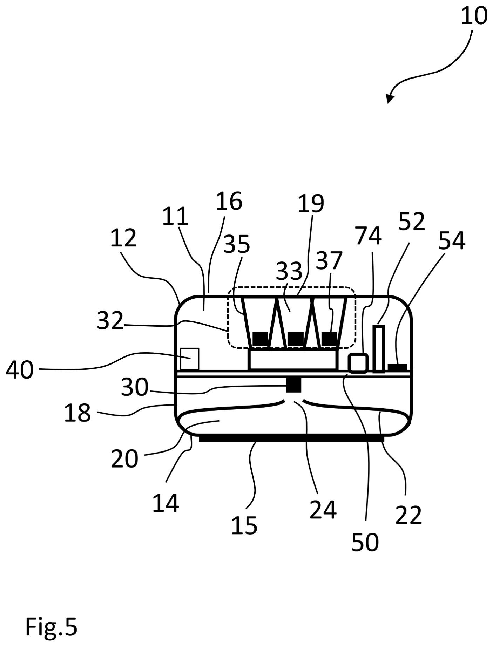

Figure 5 shows schematically a more detailed view of the chestpiece 10 and the components thereof.

The chestpiece 10 further comprises a processing unit 40.

The processing unit 40 comprises one or more processors, and one or more memory elements. A software program comprising instructions for operating the chestpiece and the components thereof is stored to the one or more memory elements.

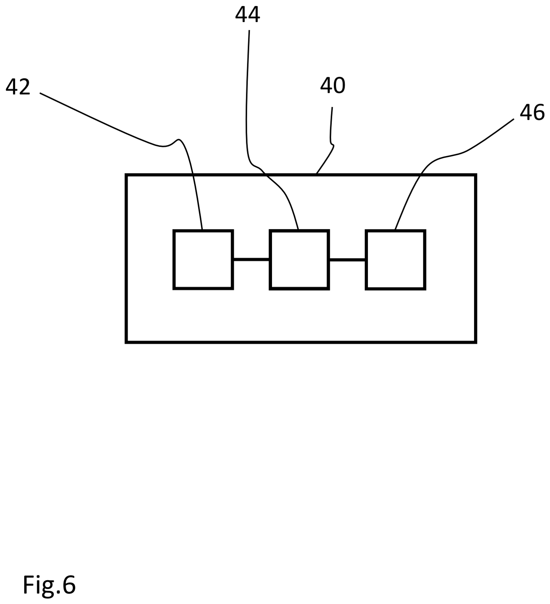

Figure 6 shows schematically the processing unit 40. The processing unit 40 comprises one or more processors 42 and a memory 44 storing instructions 46 for instructions for operating the chestpiece 10 and the components thereof.

The processing unit may comprise a read only memory (ROM) and/or a random access memory (RAM). A basic input output system (BIOS) is stored in the ROM. The RAM is used as a work area of the processing unit or as a temporary storage apparatus for loading a software program for operating the chestpiece 10 and the components thereof.

It should be noted that the above is only one embodiment, and the processing unit may be implemented in various ways without departing the present invention.

The processing unit 40 or software program stored therein may comprise instructions for processing the auscultated sound signal into auscultated sound data. The processing may comprise for example filtering and/or amplifying the auscultated sound signal and generating the auscultated sound data.

The processing unit 40 or software program stored therein is further configured to comprise instructions for operating the first sound capture module, and the auscultation microphone 30 thereof.

The processing unit 40 or software program stored therein is further configured to comprise instructions for operating the second sound capture module 32 and the ambient microphones 37 of the ambient microphone array thereof.

The chestpiece 10 further comprises a power unit 54. The power unit 54 comprises a battery. The battery is preferably a rechargeable battery.

The battery unit 54 further comprises a charging module arranged to charge the battery. The charging module is preferably provided as a wireless charging module arranged to charge the battery in wireless manner. The wireless charging module comprising a charging coil operatively connected to the battery.

The chestpiece 10 further comprises a communication module 52 configured carry out data transfer between the chestpiece 10 and an external device.

The communication module 52 is preferably a wireless communication module 52 configured to utilize a wireless communication protocol to carry out data transfer between the chestpiece 10 and the external device.

In some embodiments, the wireless communication module 52 comprises a short-range wireless communication module configured to utilize wireless short-range communication protocol to carry out the data transfer.

The wireless communication module 52 may be a Bluetooth communication module configured to utilize Bluetooth communication protocol for data transfer.

The wireless communication module 52 may also be a Wi-fi communication module configured to utilize Wi-Fi communication protocol for data transfer.

The first microphone 30 is connected or operatively connected to the processing unit 40. The processing unit 40 is configured to receive the auscultated sound signal from the first microphone 30.

The ambient microphones 37 of the microphone array of the second sound capture module 32 are connected or operatively connected to the processing unit 40. The processing unit 40 is configured to receive the ambient sound signal ambient microphones 37 of the microphone array of the second sound capture module 32.

The communication module 52 is connected or operatively connected to the processing unit 40. The communication module 52 is configured to transfer data from the chestpiece 10 or from the processing unit 40 to the external device.

The communication module 52 is further configured to receive data to the chestpiece 10 or to the processing unit 40 from the external device.

The power unit 54 is configured to provide power to the processing unit 40, the communication module 52 and to the microphones 30, 37, and possible other components.

The power unit 54 may be connected to the processing unit 40, the communication module 52 and to the microphones 30, 37, and possible other components.

The chestpiece further comprise a circuit board 50. The processing unit 40, communication module 52, power unit 54, and the microphones 30, 37 are provided to or connected to the circuit board 50.

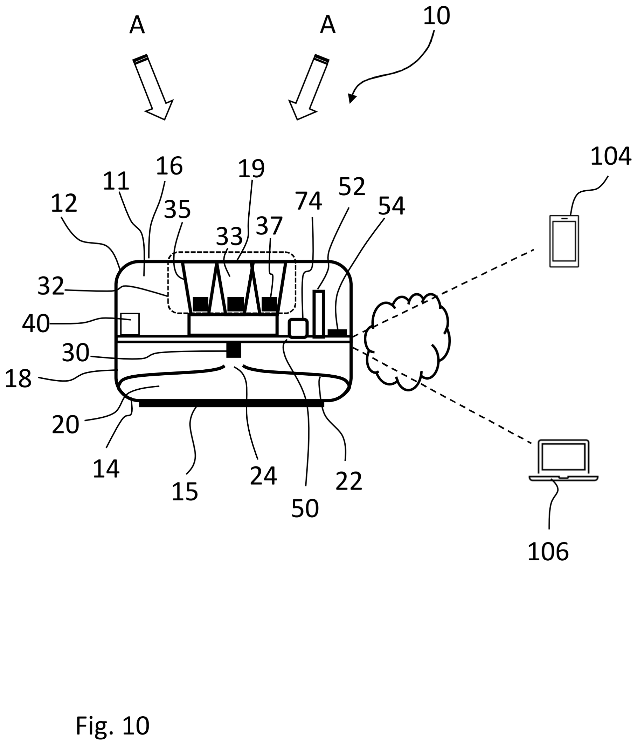

The chestpiece 10 is connected to the external device with the communication module 52 via a communication connection 100, as shown on figures 39 and 10.

The external device is a mobile user device 104 such as a module phone or a tablet. The external device may also be a computer 106 such as a laptop. The external device may also be an external server or a cloud server or the like.

The chestpiece 10 is directly connected to the external device 104, 106 via the communication module 52 by utilizing the communication protocol. Accordingly, the chestpiece 10 and the communication module 52 thereof is configured to transfer the auscultated sound signal or the auscultated sound data and/or ambient sound signal(s) and/or sound characteristics output and/or output data or other data derived from ambient sound signal and/or auscultated sound signal to the external device 104, 106.

Accordingly, the external device 104, 106 also comprises a device communication module configured to receive the auscultated sound signal or the auscultated sound data from the chestpiece 10 via the communication connection 100.

In some embodiments, the external device is an external server comprising a server communication module configured to carry out data transfer with the chestpiece 10. In this embodiment, the mobile user device 104 or the computer 106 is arranged in data transfer connection with the external server. The mobile user device 104 or the computer 106 is arranged to receive the signal data, output data or output data or other data derived from ambient sound signal and/or auscultated sound signal.

The mobile user device 104 or the computer 106 is configured to emit the auscultated sounds of the auscultated signal with a speaker device provided to the mobile user device 104 or the computer 106. Accordingly, chestpiece 10 enables the auscultated sounds to be listened on site where the patient is or at distant location over the communication connection 100.

The chestpiece 10 may further comprise a movement sensor 74, or one or more movement sensors 74.

The movement sensor 74 is an accelerometer, gyroscope or magnetometer, or some other movement sensor or a combination thereof.

The movement sensor 74 is configured to detect movement of the chestpiece 10.

The movement sensor 74 is configured to detect movement of the chestpiece 10 and generate a movement signal based on the detected movement of the chestpiece 10.

The movement sensor 74 is connected to the processing unit 40. The processing unit 40 is configured to the receive the movement signal(s) from the movement sensor 74.

The movement sensor 74 may also be omitted.

The processing unit 40 of the chestpiece 10 is configured to selectively operate the chestpiece 10 in an auscultation mode and in an ambient mode.

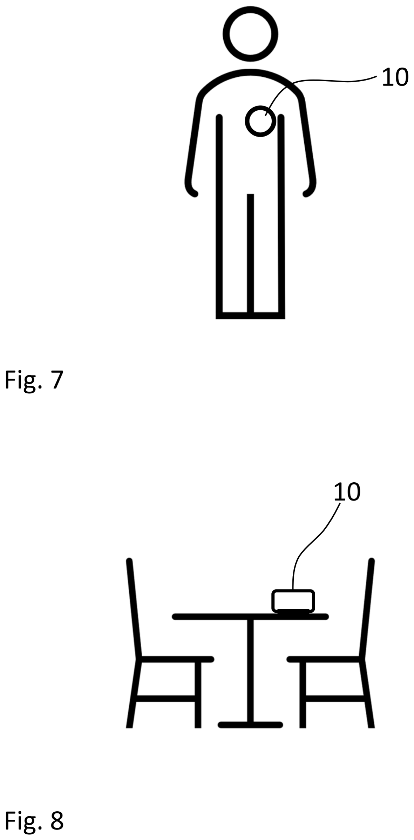

Figure 7 shows schematically the auscultation mode in which the first sound capture module 15, 20, 30 is configured to capture internal body sounds of the patient body in contact with the patient body via the contact surface 14, 15 and to convert the internal body sounds into an internal body sound signal. In the auscultation mode contact surface 14, 15 is paced on the body of the patient.

Figure 8 shows schematically the ambient mode in which the second sound capture module 32 is configured to capture ambient sounds from the ambient space of the chestpiece 10 and to convert the ambient sounds into the ambient sound signals. In the ambient mode the chestpiece 10 is usually placed on a support surface and is immobile such that the second sound capture module 32 is enabled to capture ambient sounds.

The present inventio provides an auscultation system comprising an auscultation sound processing module and an ambient sound processing module.

The auscultation sound processing module is configured to perform processing of the auscultation sound signal captured by the first sound capture module and the first microphone thereof. The auscultation sound processing module is configured receive the auscultation sound signal from the first microphone 30 and generate an auscultation audio output.

In some embodiments, the auscultation sound processing module is configured to receive the ambient sound signal from the second sound capture module 32 and perform noise cancellation to the auscultation sound signal by utilizing the ambient sound signal.

Accordingly, in the auscultation mode the processing unit 40 of the chestpiece is configured operate and activate both the first microphone 30 of the first sound capture module and the microphones 37 of the microphone array of the second sound capture module 32, or alternative only the first microphone 30 of the first sound capture module.

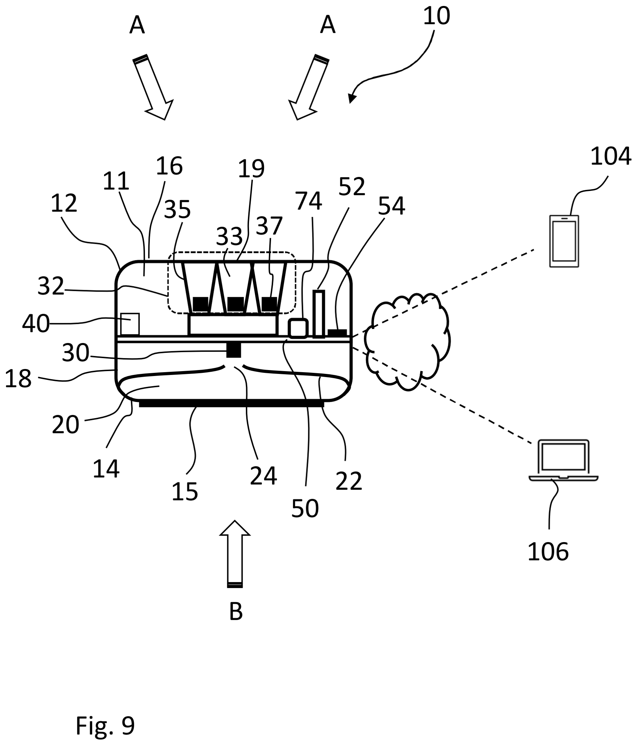

Figure 9 shows schematically the auscultation mode in which the first sound capture module 15, 20, 30 is configured to capture internal body sounds B of the patient body in contact with the patient body via the contact surface 14, 15 and to convert the internal body sounds into an internal body sound signal.

In the auscultation mode the second sound capture module 32 is configured to capture ambient sounds A from ambient space of the chestpiece 10.

The auscultation sound processing module may be provided to the chestpiece 10, and operatively connected to the processing unit 40 or provided in connection with processing unit 40. Alternatively, auscultation sound processing module is provided to an external computing device 104, 106 configured to receive the auscultation sound signal, or the auscultation sound signal and the ambient sound signal from the chestpiece 10. Further alternatively, auscultation sound processing module is provided as a distributed module configured to distribute the processing of the auscultation sound signal, or the auscultation sound signal and the ambient sound signal between the chestpiece 10 and one or more external computing devices 104, 106, or between two or more external devices 104, 106.

The ambient sound processing module 200 is configured to perform processing of the ambient sound signals or the combined single ambient sound signal outputted from the second sound capture module 32.

The ambient sound processing module 200 is configured to receive the ambient sound signal(s) from the second sound capture module 32.

Accordingly, in the ambient mode the processing unit 40 of the chestpiece is configured operate and activate the microphones 37 of the microphone array of the second sound capture module 32.

Further, in the ambient mode the processing module 200 is configured to disable operation of the first microphone 30. Alternatively, the ambient sound processing module 200 does not receive or utilize the auscultation sound signal from the first microphone 30.

In the ambient mode the ambient sound processing module 200 is configured to obtain the ambient sound signal of the second sound capture module 32, carrying out audio feature extraction to the ambient sound signal to obtain extracted audio features from the ambient sound signal, generating audio feature data of the ambient sound signal based on the extracted audio features, and obtaining a sound characteristic output by processing the audio feature data of the ambient sound signal.

The auscultation system or the ambient sound processing module 200 comprises an output unit configured to provide the sound characteristic output as an ambient sound analysis output.

The ambient sound processing module 200 may be configured to generate a visual or audio output data upon obtaining the sound characteristic output such that the sound characteristic output comprises the visual or audio output data.

The auscultation system further comprises the external device, such as the external mobile user device 104 or the computer 106.

The ambient sound processing module 200 may be configured to transmit the sound characteristic output to the external user device 104, 106.

The external device 102, 106 is configured to receive the sound characteristic output.

The external device 102, 106 is configured to present the visual or audio output data of the sound characteristic output.

Figure 10 shows schematically the ambient mode in which the second sound capture module 32 is configured to capture ambient sounds A from the ambient space of the chestpiece 10 and to convert the ambient sounds into the ambient sound signal(s).

In the auscultation mode the second sound capture module 32 is configured to capture ambient sounds A from ambient space of the chestpiece 10.

The ambient sound processing module 200 comprises one or more processors, and one or more memory elements. A software program comprising instructions for carrying out processing the ambient sound signal(s).

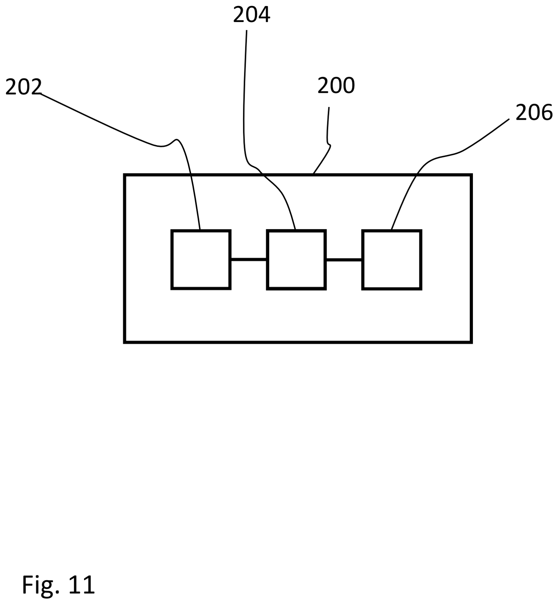

Figure 11 shows schematically the ambient sound processing module 200. The ambient sound processing module 200 comprises one or more processors 202 and a memory 204 storing instructions 206 for instructions for carrying out processing the ambient sound signal(s).

The ambient sound processing module 200 is provided to the chestpiece 10, and operatively connected to the processing unit 40 or provided in connection with processing unit 40. Alternatively, the ambient sound processing module 200 is provided to an external computing device 104, 106 configured to receive the ambient sound signal from the chestpiece 10. Further alternatively, the ambient sound processing module 200 is provided as a distributed module configured to distribute the processing of the ambient sound signal between the chestpiece 10 and one or more external computing devices 104, 106, or between two or more external devices 104, 106.

In some embodiments, the processing unit 40 is configured to initiate the ambient mode as response to detecting immobility of the chestpiece 10 with the movement sensor 74, or initiate the ambient mode as response to detecting immobility of the chestpiece 10 a pre-determined immobility time period with the movement sensor 74, such as 1 minute or 5 minutes. The processing unit is further configured to terminate the ambient mode as response to detecting movement of the chestpiece 10 with the movement sensor 74.

The present invention provides a computer-implemented method executed by the ambient sound processing module 200, the method causing the ambient sound processing module 200 the processing of the ambient sound signal(s).

The computer implemented method comprises
- obtaining the ambient sound signal of the second sound capture module 32,
- carrying out audio feature extraction to the ambient sound signal to obtain extracted audio features from the ambient sound signal,
- generating audio feature data of the ambient sound signal based on the extracted audio features, and
- obtaining a sound characteristic output by processing the audio feature data of the ambient sound signal.

The method may also comprise pre-processing of the ambient sound signal.

In some embodiments, the pre-processing comprises high-pass filtering. The high-pass filtering is configured to remove low-frequency noise by applying a high-pass filter. The high-pass filter is configured to remove sounds having frequency below 100 Hz or below 150 Hz. This ensures retention of higher frequencies essential for further speech, breathing and cough analysis, eliminating rumble and environmental noise. The high-pass filtering may be carried out by a high-pass filtering algorithm.

The pre-processing may also comprise carrying out automatic gain control (AGC). The automatic gain control is configured to adjust input levels of the ambient microphones 37 dynamically. This ensures consistent audio capture across varying sound intensities, preventing clipping during loud coughs and maintaining sensitivity to softer sounds. The automatic gain control may be carried out by an automatic gain control algorithm.

The method may also comprise post-processing of the ambient sound signal carried out before the audio feature extraction.

The post-processing comprises utilizing speech enhancement algorithms, such as spectral subtraction and Wiener filtering, specifically designed to remove residual background noise without compromising vocal quality.

In some embodiments comprise carrying out voice activity detection (VAD) configured to refine the ambient sound signal by identifying segments of relevant sounds, such as speech, breathing coughs or external body sounds, for focused processing. This enables determining and isolating human originating sound components of the ambient sound signal from ambient noise and reduces overall data for further analysis. The voice activity detection may further comprise identification and classification of different human originating sound components from each other.

The voice activity detection may be carried out by utilizing machine learning algorithms, such as CNNs and RNNs, trained on a variety of audio samples, identification and classification of human originating sound components. The algorithm may also be configured to isolate different human originating sound components from each other.

Based on the above, the method may comprise determining human originating sound components in the ambient sound signal by utilizing the voice activity detection. The method then further comprises carrying out the audio feature extraction to the detected human originating sound components to obtain extracted audio features from the detected human originating sound components, generating the audio feature data of the detected human originating sound components based on the extracted audio features, and obtaining the sound characteristic output by processing the audio feature data of the detected human originating sound component.

Determining the human originating sound components in the ambient sound signal may comprise one or more of the following:
- identifying speech sound components in the ambient sound signal, the speed sound components representing sound components originating from human speech, or
- identifying cough sound components in the ambient sound signal, the cough sound components representing sound components originating from human cough, or
- identifying external body sound components in the ambient sound signal, the external body sound components representing sound components originating from human body; or
identifying breathing sound components in the ambient sound signal, the breathing sound components representing sound components originating from human breathing.

For carrying out further analysis of the ambient sound signal, a feature extraction is a crucial step. The objective of the feature extraction is to transform raw ambient sound signal(s) into specific, measurable characteristics (technical features) that reveal valuable insights about the sound. Different categories of technical features provide distinct technical details of the ambient sound signal. The extracted technical features of the ambient sound signal are used for further processing or analysis of the ambient sound signal.

The method comprises carrying out audio feature extraction to the ambient sound signal or the detected human originating sound components to obtain extracted audio features. The audio feature extraction may comprise one or more of the following:
- time-domain feature extraction configured to determine intensity features of the ambient sound signal over time,
- frequency-domain feature extraction configured to determine frequency features of the ambient sound signal,
- cepstral feature extraction configured to determine vocal track shape features of the ambient sound signal,
- voice quality feature extraction configured to determine consistency or smoothness or consistency and smoothness features of the ambient sound signal,
- temporal feature extraction configured to determine pattern features of the ambient sound signal, and
- prosodic feature extraction configured to determine expressiveness features of the ambient sound signal.

Time-domain features define the amplitude of the sound wave over time. They capture basic information about the signal's intensity and patterns, providing foundational data for detecting voice activity or recognizing certain sound types. The time-domain features may comprise one or more of the following:
- Amplitude Envelope which is configured to determine changing volume levels in the ambient sound signal, highlighting where sound intensities increase or decrease. This is useful for identify strong sounds like coughs or laughter,
- Zero-Crossing Rate which is configured to determine how often the audio signal crosses the zero amplitude level. This is usually associated with high-pitched or fricative sounds, such as "s" and "f" sounds, and
- Signal Energy which is configured to determine the power of the ambient sound signal. This enables to differentiate between speech and background noise.

The frequency-domain features provide insight into the tonal quality of the audio by breaking down the ambient sound signal into frequency components. These features are important for example in speech recognition, speaker identification. The frequency-domain features may comprise one or more of the following:
- Fundamental Frequency (Pitch) is configured to determine lowest frequency in the ambient sound signal, or in speech. This provides insight to vocal fold vibration and speaker identity. This further provides information of intonation and is frequently utilized in emotion detection, as pitch fluctuations correlate with emotional expressions,
- Formants is configured to determine resonant frequencies in the vocal tract that shape vowel sounds and enable distinguish different speech sounds. Formants provides insights into articulation and phonation issues of speech, and
- Spectral Centroid is configured to determine center of mass of audio spectrum in the ambient sound signal. This provides indication of the "brightness" or "sharpness" of a sound. This further enables distinguishing clearer speech from muffled audio.

The cepstral features analyze the ambient sound signal in a way that focuses on vocal tract shape, which is key to capturing phonetic information and identifying speaker traits. The cepstral features may comprise one or more of the following:
- Mel-Frequency Cepstral Coefficients (MFCCs) is configured to determine the shape of the spectral envelope. This may be used in speech and speaker recognition due to closely model human auditory perception, and
- Cepstral Peak Prominence is configured determine clarity of pitch by analyzing the amplitude of the cepstral peaks. This enables differentiating between clear speech and breathy or hoarse sounds, which may indicate vocal fatigue or health issues.

The voice quality features focus on the consistency and smoothness of the vocal signal. This may be associated with health and emotional states. The voice quality features may comprise one or more of the following:
- Harmonic-to-Noise Ratio (HNR) is configured to determine the ratio between harmonic (structured) components and noise in the signal. This may indicate vocal strain or pathology,
- Jitter and Shimmer is configured to determine the variability in pitch (jitter) and loudness (shimmer). These are indicators of voice stability. Variations can be linked to emotional arousal, stress, and clinical conditions affecting the vocal cords, and
- Glottal Flow Characteristics is configured determine the airflow pattern through the vocal folds. This provides further insight into voice quality and helping identify issues like breathiness or harshness.

The temporal features measure patterns over time, revealing speech rhythm and structure. This enabled linguistic and emotion-based analysis. The temporal features may comprise one or more of the following:
- Duration of Speech Segments is configured to determine the length of spoken parts, pauses, and silences. This provides insight into the speaking rate and fluency, which can be linked to cognitive or emotional states,
- Pause Patterns is configured to determine natural breaks in speech. This indicates emphasis and helping in sentiment analysis, as longer pauses may suggest hesitation or thoughtfulness, and
- Speech Rate is configured to determine speed of speech. This may reflect emotional excitement, calmness, or nervousness.

The prosodic features capture the expressiveness in speech. This is often tied to the speaker's emotional tone or communicative intent. The prosodic features may comprise one or more of the following:
- Intonation is configured to determine pitch variations across phrases and sentences. This enables conveying emotion and emphasis. Rising and falling tones can indicate questions, statements, or affective states,
- Stress is configured to determine certain words or syllables, which plays a role in conveying intent or emotional intensity, and
- Rhythm is configured to determine timing and cadence of speech, helping to distinguish natural conversational speech from monotonic or robotic patterns.

The method may comprise carrying out one or more above disclosed audio feature extractions or extraction steps. Different combinations of the above audio feature extractions may be carried out for different purposes for obtaining different sound characteristic outputs.

The audio feature extractions, as disclosed above, may be carried out by utilizing machine learning algorithms, such as CNNs and RNNs, trained on a variety of audio samples.

In some embodiments, the method comprises obtaining the sound characteristic output by obtaining speech analysis output by processing the audio feature data generated by carrying out one or more of the following: prosodic feature extraction, voice quality feature extraction, a temporal feature extraction for health monitoring of patient. This enables monitoring emotional states, cognitive health and neurological conditions of the patient by carrying out the speech analysis based on the extracted audio features.

For example, voice quality features, such as harmonic-to-noise ratio (HNR) and glottal flow characteristics, can reflect vocal health, which is often influenced by physical conditions. A breathy or strained voice might indicate vocal fatigue, dehydration, or even respiratory health issues.

In some embodiments, method comprises obtaining the sound characteristic output by obtaining speech analysis output by processing the audio feature data generated by carrying out prosodic feature extraction and voice quality feature extraction for emotional state monitoring of a patient.

For example, prosodic features, such as intonation, rhythm, and pitch variations, can reveal emotional tones like stress, calmness, or agitation. Jitter, shimmer, and pitch patterns are also useful here; increased jitter or shimmer, along with fluctuating pitch, can suggest emotional strain or anxiety.

In some embodiments, the method comprises obtaining the sound characteristic output comprises obtaining speech analysis output by processing the audio feature data generated by carrying out temporal feature extraction for neurological health monitoring of a patient.

Temporal features like speech rate, pause patterns, and duration of speech segments are particularly relevant in detecting cognitive decline, as often observed in conditions like Parkinson's or Alzheimer's disease. Reduced speech rate, prolonged pauses, or irregular rhythm can indicate cognitive impairment or neurological fatigue. Additionally, abnormalities in formant patterns may reveal issues with articulation and vocal control.

The method may comprise identifying speech sound components in the ambient sound signal and carrying out the audio feature extraction to the identified speech sound components. The method further comprises generating a speech audio feature data based on the extracted audio features and obtaining speech sound characteristic output by processing generating a speech audio feature data of the speech sound components. The speech sound characteristic output representing speech analysis output.

Cough sounds are also rich in information about a person's respiratory health and can reflect conditions from common colds to chronic respiratory diseases.

In some embodiments, the method comprises obtaining the sound characteristic output by obtaining cough analysis output by processing the audio feature data generated by carrying out one or more of the following: time-domain feature extraction, frequency-domain feature extraction and cepstral feature extraction for cough monitoring of a patient. This may provide information of respiratory health of the patient.

In some embodiments, the method comprises obtaining the sound characteristic output comprises obtaining cough analysis output by processing the audio feature data generated by carrying out frequency-domain feature extraction and cepstral feature extraction for cough classification of patient.

Frequency-domain features like spectral centroid and cepstral features such as Mel-Frequency Cepstral Coefficients (MFCCs) enable distinguishing different types of coughs, such as wet versus dry coughs. A wet cough, characterized by lower frequency components, can signal respiratory infections or mucus in the airways, while a dry cough with higher frequencies may indicate conditions like asthma or bronchitis.

In some embodiments, the method comprises obtaining the sound characteristic output comprises obtaining cough analysis output by processing the audio feature data generated by carrying out time-domain feature extraction for cough characteristics monitoring.

Time-domain features, including amplitude envelope and signal energy, allow for the assessment of cough strength and frequency. By analyzing the duration and energy of cough events, the system can help track the progression of conditions like chronic obstructive pulmonary disease (COPD) or identify exacerbations in asthma patients.

The method may comprise identifying cough sound components in the ambient sound signal and carrying out the audio feature extraction to the identified cough sound components. The method further comprises generating a cough audio feature data based on the extracted audio features and obtaining cough sound characteristic output by processing generating a cough audio feature data of the cough sound components. The cough sound characteristic output representing cough analysis output.

Breathing sounds are also rich in information about a person's respiratory health and can reflect conditions from common colds to chronic respiratory diseases. Furthermore. breathing sounds enable motoring or tracking sleep of the person.

In some embodiments, the method comprises obtaining the sound characteristic output by obtaining breathing analysis output by processing the audio feature data generated by carrying out one or more of the following: time-domain feature extraction, frequency-domain feature extraction, cepstral feature extraction, temporal feature extraction and voice quality feature extraction for breathing monitoring of a patient. This may provide information of respiratory health of the patient and/or information of sleep of the patient.

In some embodiments, the method comprises obtaining the sound characteristic output comprises obtaining breathing analysis output by processing the audio feature data generated by carrying out frequency-domain feature extraction and temporal feature extraction for breathing classification of patient.

In some embodiments, the method comprises obtaining the sound characteristic output comprises obtaining breathing analysis output by processing the audio feature data generated by carrying out frequency-domain feature extraction and temporal feature extraction for breathing classification of patient.

Frequency-domain features like spectral centroid and cepstral features such as Mel-Frequency Cepstral Coefficients (MFCCs) enable distinguishing different types of breathing.

In some embodiments, the method comprises obtaining the sound characteristic output comprises obtaining breathing analysis output by processing the audio feature data generated by carrying out time-domain feature extraction for breathing characteristics monitoring.

Time-domain features, including amplitude envelope and signal energy, allow for the assessment of breathing strength and frequency. By analyzing the duration and energy of breathing events, the system can help track the progression of conditions.

The method may comprise identifying breathing sound components in the ambient sound signal and carrying out the audio feature extraction to the identified breathing sound components. The method further comprises generating a breathing audio feature data based on the extracted audio features and obtaining breathing sound characteristic output by processing generating a breathing audio feature data of the breathing sound components. The breathing sound characteristic output representing breathing analysis output.

The invention has been described above with reference to the examples shown in the figures. However, the invention is in no way restricted to the above examples but may vary within the scope of the claims.

## Claims

1. A chestpiece (10) of an electronic stethoscope for auscultation and sound analysis, the chestpiece (10) comprising:
- a body (12),
- a first sound capture module (15, 20, 30) in connection with the body (12), the first sound capture module (15, 20, 30) comprising a contact surface (14, 15) for contact with a patient body, and being configured to capture internal body sounds in contact with the patient body via the contact surface (14 15) and to convert the internal body sounds into an internal body sound signal, and
- a second sound capture module (32, 37) in connection with the body (12), the second sound capture module (32, 37) being configured to capture ambient sounds from ambient space of the chestpiece (10) and to convert the ambient sounds into an ambient sound signal, and
- a processing unit (40, 42, 44, 46) for operating the chestpiece (10),
**characterized in that** the second sound capture module (32) comprises three or more ambient microphones (37) arranged spaced apart from each other and configured to capture ambient sounds from ambient space of the chestpiece (10) and to convert the ambient sounds into the ambient sound signals.

2. A chestpiece (10) according to claim 1, **characterized in that**:
- the second sound capture module (32) comprises three or more separate ambient microphones (37) arranged spaced apart from each other and configured to capture ambient sounds from ambient space of the chestpiece (10) and to convert the ambient sounds into the ambient sound signals; or
- the second sound capture module (32) comprises six or more separate ambient microphones (37) arranged spaced apart from each other and configured to capture ambient sounds from ambient space of the chestpiece (10) and to convert the ambient sounds into the ambient sound signals.

3. A chestpiece (10) according to claim 1, **characterized in that**:
- the second sound capture module (32) comprises a microphone array comprising three of more ambient microphones (37) arranged spaced apart from each other and configured to capture ambient sounds from ambient space of the chestpiece (10) and to convert the ambient sounds into the ambient sound signals; or
the second sound capture module (32) comprises a microphone array comprising six or more ambient microphones (37) arranged spaced apart from each other and configured to capture ambient sounds from ambient space of the chestpiece (10) and to convert the ambient sounds into the ambient sound signals.

4. A chestpiece (10) according to any one of claims 1 to 3, **characterized in that** the processing unit (40, 42, 44, 46) is configured to selectively operate the chestpiece (10) in an auscultation mode and in an ambient mode, and that
in the auscultation mode:
the first sound capture module (15, 20, 30) is configured to capture internal body sounds of the patient body in contact with the patient body via the contact surface (14 15) and to convert the internal body sounds into an internal body sound signal; and
in the ambient mode:
the second sound capture module (32) is configured to capture ambient sounds from the ambient space of the chestpiece (10) and to convert the ambient sounds into the ambient sound signals.

5. A method for sound analysis, the method utilizing a chestpiece (10) for auscultation, the chestpiece (10) comprising:
a body (12),
a first sound capture module (15, 20, 30) in connection with the body (12), the first sound capture module (15, 20, 30) comprising a contact surface (14, 15) for contact with a patient body, and being configured to capture internal body sounds in contact with the patient body via the contact surface (14 15) and to convert the internal body sounds into an internal body sound signal, and
a second sound capture module (32) in connection with the body (12), the second sound capture module (32) being configured to capture ambient sounds from ambient space of the chestpiece (10) and to convert the ambient sounds into an ambient sound signal; and
the method being executed using hardware and software resources of computing device, the method causing the computing device to perform operations comprising:
- obtaining the ambient sound signal from the second sound capture module (32),
- carrying out audio feature extraction to the ambient sound signal to obtain extracted audio features from the ambient sound signal,
- generating audio feature data of the ambient sound signal based on the extracted audio features, and
- obtaining a sound characteristic output by processing the audio feature data of the ambient sound signal.

6. A method according to claim 5, **characterized in that** carrying out audio feature extraction to obtain extracted audio features from the ambient sound signal comprises one or more of the following:
- time-domain feature extraction configured to extract intensity features of the ambient sound signal over time,
- frequency-domain feature extraction configured to extracting frequency features of the ambient sound signal,
- cepstral feature extraction configured to extract vocal track shape features of the ambient sound signal,
- voice quality feature extraction configured to extract consistency or smoothness or consistency and smoothness features of the ambient sound signal,
- temporal feature extraction configured to extract pattern features of the ambient sound signal, and
- prosodic feature extraction configured to extract expressiveness features of the ambient sound signal.

7. A method according to claim 5 or 6, **characterized in that** the method comprises
- determining human originating sound components in the ambient sound signal,
- carrying out the audio feature extraction to the detected human originating sound components to obtain extracted audio features from the detected human originating sound components,
- generating the audio feature data of the detected human originating sound components based on the extracted audio features, and
- obtaining the sound characteristic output by processing the audio feature data of the detected human originating sound component.

8. A method according to claim 7, **characterized in that** determining human originating sound components in the ambient sound signal comprises:
- identifying speech sound components in the ambient sound signal, the speed sound components representing sound components originating from human speech, or
- identifying cough sound components in the ambient sound signal, the cough sound components representing sound components originating from human cough, or
- identifying external body sound components in the ambient sound signal, the external body sound components representing sound components originating from human body; or
- identifying breathing sound components in the ambient sound signal, the breathing sound components representing sound components originating from human breathing.

9. A method according to any one of claims 5 to 8, **characterized in that** the method is carried out by utilizing the chestpiece (10) according to any one of claims 1 to 4.

10. A method according to any one of claims 5 to 9, **characterized in that** the method is carried out by utilizing the auscultation system according to any one of claims 11 to 12.

11. An auscultation system for auscultation and sound analysis, the auscultation system comprises:
- a chestpiece (10) for auscultation, the chestpiece (10) comprising:
a body (12),
a first sound capture module (15, 20, 30) in connection with the body (12), the first sound capture module (15, 20, 30) comprising a contact surface (14, 15) for contact with a patient body, and being configured to capture internal body sounds in contact with the patient body via the contact surface (14 15) and to convert the internal body sounds into an internal body sound signal, and
a second sound capture module (32) in connection with the body (12), the second sound capture module (32) being configured to capture ambient sounds from ambient space of the chestpiece (10) and to convert the ambient sounds into an ambient sound signal, and
a processing unit (40, 42, 44, 46) for operating the chestpiece (10),
**characterized in that** the second sound capture module (32) comprises a microphone array comprising three of more ambient microphones (37) arranged spaced apart from each other and configured to capture ambient sounds from ambient space of the chestpiece (10) and to convert the ambient sounds into the ambient sound signals, and that
the auscultation system further comprises:
- an ambient sound processing module (200) configured to perform processing of the ambient sound signals, the processing comprising:
obtaining the ambient sound signal from the second sound capture module (32),
carrying out audio feature extraction to the ambient sound signal to obtain extracted audio features from the ambient sound signal,
generating audio feature data of the ambient sound signal based on the extracted audio features, and
obtaining a sound characteristic output by processing the audio feature data of the ambient sound signal; and
- an output unit configured to provide the sound characteristic output as an ambient sound analysis output.

12. An auscultation system according to claim 11, **characterized in that** the processing unit (40, 42, 44, 46) is configured to selectively operate the chestpiece (10) in an auscultation mode and in an ambient mode, and that
in the auscultation mode:
the first sound capture module (15, 20, 30) is configured to capture internal body sounds of the patient body in contact with the patient body via the contact surface (14 15) and to convert the internal body sounds into an internal body sound signal, and
in the ambient mode:
the second sound capture module (32) is configured to capture ambient sounds from the ambient space of the chestpiece (10) and to convert the ambient sounds into the ambient sound signal, and the ambient sound processing module (200) configured to perform the processing of the ambient sound signals.

13. An auscultation system according to claim 11 or 12, **characterized in that** the chestpiece (10) is a chestpiece according to the any one of claims 1 to 4.

14. An auscultation system according to any one of claims 11 to 13, **characterized** in that- the auscultation system is configured to carry out the method according to any one claims 5 to 10.

15. A computer program product comprising computer instructions in at least one memory which, when executed by at least one processor, cause a computing apparatus to perform the method according to any one of claims 5 to 10.
